# EUROPÄISCHE PATENTSCHRIFT

(11) **EP 2 196 255 B1**
(45) Veröffentlichungstag und Bekanntmachung des Hinweises auf die Patenterteilung: **24.10.2012**
(21) Anmeldenummer: 10003411.5
(22) Anmeldetag: 22.11.2007
(51) Int. Cl.: B01F 3/14, B01F 7/16, B01F 15/02, B29B 7/94, B29C 47/10

(54) **Verfahren und Vorrichtung zur Einbringung von Zusatzstoffen**
Method and device for introducing additive materials
Procédé et dispositif d'introduction d'additifs

(30) Priorität: 23.11.2006 AT 19512006
(43) Veröffentlichungstag der Anmeldung: 16.06.2010
(62) Teilanmeldung aus: 07815193.3
(73) Patentinhaber: EREMA Engineering Recycling Maschinen und Anlagen Gesellschaft m.b.H., 4052 Ansfelden (AT)
(72) Erfinder: Wendelin, Gerhard, 4030 Linz (AT); Hackl, Manfred, 4040 Linz-Urfahr (AT); Feichtinger, Klaus, 4040 Linz (AT)
(74) Vertreter: Wildhack, Andreas

(56) Entgegenhaltungen:
- DE-A1- 2 134 305
- DE-U1- 20 011 402
- GB-A- 728 323
- GB-A- 778 953

## Beschreibung

Die Erfindung betrifft ein Verfahren gemäß dem Oberbegriff des Anspruchs 1 sowie eine Vorrichtung zur Durchführung des Verfahrens gemäß dem Oberbegriff des Anspruchs 8.

Aus dem Stand der Technik sind zahlreiche Verfahren und Vorrichtungen bekannt, bei denen die flüssigen Zusatzstoffe entweder von oben auf das Kunststoffmaterial aufgesprüht werden oder die Zugabe im Wirbelschichtverfahren erfolgt.

Aus der US 4,522,957 ist es bekannt, flüssige Zusatzstoffe zu Kunststoffgranulat in einem Mischer hinzuzufügen.

In der WO 00/38895 wird ein derartiges Verfahren zur Verminderung der Staubbelastung bzw. Staubentstehung weiter verbessert, indem in einem ersten Schritt der flüssige Zusatzstoff in einer Sprühkammer auf die Plastikgranulate im Gegenstromverfahren aufgedüst wird und anschließend ein statisches Mischverfahren erfolgt.

In der EP 7624 wird ein flüssiger Zusatzstoff zu Kunststoffgranulat zugesetzt, und zwar in einem inerten Gasstrom.

In der WO 84/02530 werden die Kunststoffgranulate zuerst in einem Durchlaufmischer verwirbelt und werden in diesem verwirbelten Zustand im Gasstrom mit hocherhitztem flüssigen Zusatzstoff benetzt.

Aus der WO 9425509 ist ein Verfahren bekannt, bei dem ein Polymergranulat in einer Mischeinrichtung mit einem flüssigen Additiv über eine Einspritzdüse benetzt wird, wobei zur besseren Benetzung die Oberfläche des Kunststoffgranulates unregelmäßig strukturiert bzw. aufgeraut ist.

In der WO 2006/010291 wird ein Verfahren und eine Mischvorrichtung beschrieben, bei denen ein flüssiges Additiv über eine Einspritzvorrichtung in einer Mischeinrichtung einem Kunststoffgranulat zugedüst wird und danach die Mischung in einen Extruder gelangt.

Weiters ist aus der EP 9817 ein Verfahren bekannt, bei dem Kunststoffgranulat zuerst mit einem "coupling agent" bzw. Träger benetzt wird, der eine bessere Verteilung der flüssigen Additive auf der Oberfläche des Kunststoffgranulats sicherstellen soll. Als coupling agents werden insbesondere Paraffine bzw. paraffinartige Substanzen angeführt.

Weiters ist aus der US 4,703,093 ein Verfahren bekannt, bei dem ein flüssiger Zusatzstoff zu einem bereits vorgewärmten Kunststoffgranulat zugesetzt wird.

Die DE 263 16 22 beschreibt ein Verfahren zum gleichzeitigen und kontinuierlichen Zuführen von pulverförmigen Feststoffen und Flüssigkeiten in Behandlungsmaschinen. Dies geschieht über eine Ringdüse, wobei die Flüssigkeit zu einem schlauchartigen Mantel geformt wird, in dessen Zentrum die Feststoffe eingegeben werden.

Derartige Verfahren sind jedoch in erster Linie nur für dünnflüssige, fein zerstäubbare Zusatzstoffe geeignet und funktionieren für höherviskose, zähflüssige Zusatzstoffe oder für Zusatzstoffe von fester oder halbfester Konsistenz nur ungenügend. Meist wird das Kunststoffmaterial nur unvollständig und ungleichmäßig benetzt.

Werden höherviskose Zusatzstoffe auf höhere Temperaturen erhitzt, um dennoch in dünnflüssiger Form zugegeben werden zu können, so bilden sich an kühleren Stellen oder kälteren Oberflächen der Vorrichtung oft Ablagerungen oder Niederschläge der Zusatzstoffe aus.

Dies führt zu Schwierigkeiten und Ungenauigkeiten bei der Dosierung und zur Verschmutzung der Vorrichtungen.

DE 2 134 305 A offenbart ein Verfahren gemäß dem Oberbegriff des Anspruchs 1 und eine Vorrichtung gemäß dem Oberbegriff des Anspruchs 8.

Aufgabe der Erfindung ist es, ein Verfahren und eine Vorrichtung zu schaffen, durch das/die nicht-trocken-teilchenförmige, höherviskose Zusatzstoffe zu einem vorgelegten stückigen Material, insbesondere einem Kunststoffmaterial bzw. von Polymerteilchen, einfach und gleichmäßig zugegeben werden können. Die Oberfläche des Materials soll dabei möglichst vollständig und gleichmäßig mit den Zusatzstoffen benetzt werden und die Zusatzstoffe sollen innerhalb der Materialteilchen gleichmäßig verteilt bzw. dispergiert werden. Außerdem sollen die Zusatzstoffe in korrekten Dosierungen zugegeben und Ablagerungen und somit Verschmutzungen an ungewünschten Stellen vermieden werden können.

Diese Aufgaben werden durch die kennzeichnenden Merkmale der Ansprüche 1 bzw. 8 gelöst.

Durch das erfindungsgemäße Verfahren bzw. durch die erfindungsgemäße Vorrichtung wird es ermöglicht, Zusatzstoffe bzw. Beschichtungsstoffe in vorteilhafter Weise auf stückige, teilchenförmig vorliegende Materialien sehr gleichmäßig und homogen aufzubringen, sodass sich eine vollständige Benetzung der Oberfläche der Materialteilchen ergibt.

Außerdem können so auch geringste Mengen an Zusatzstoffen genau dosiert werden, da die gesamte Menge der eingesetzten Zusatzstoffe direkt in die Materialteilchen eingebracht wird und es für die Zusatzstoffe keine Möglichkeit gibt, sich abzulagern. Dies wird insbesondere dadurch gewährleistet, dass die Zusatzstoffe mit keinen kälteren Bauteilen des Behälters bzw. Reaktors in Berührung kommen. Es kommt es zu keinerlei Verschmutzungen oder Materialablagerungen von kondensierten bzw. erstarrten Zusatzstoffen an unerwünschten Stellen im Reaktor, wodurch eine oftmalige Reinigung nicht mehr notwendig ist. Der Niederschlag von Zusatzstoffen oder von Staub mit Zusatzstoffen an kühleren Stellen wird im Vergleich zu der aus dem Stand der Technik bekannten Eindosierung durch Aufsprühen der Zusatzstoffe auf die Materialteichen von oben stark verringert oder sogar verhindert.

Die dynamische Bewegung bzw. Rotation der Materialteilchen im Behälter erleichtert die Einbringung der Zusatzstoffe, das Aufbringen auf die Oberflächen der Materialteilchen und unterstützt die gleichmäßige Verteilung bzw. Dispergierung der Zusatzstoffe auf die Materialteilchen. Dies wird dadurch gewährleistet, dass die Materialteilchen an der Innenseite der Seitenwand des Behälters entlanggleiten bzw. vorbeirotieren und auf diese Weise die dort austretenden Zusatzstoffe mitnehmen bzw. mitreißen.

Durch das erfindungsgemäße Verfahren wird somit die gesamte Oberfläche benetzt und die Zusatzstoffe in der Mischung der Teilchen optimal verteilt.

Weitere Vorteile der Erfindung finden sich in den abhängigen Ansprüchen.

Es können eine oder mehrere Zugabeeinrichtungen vorgesehen sein. Diese Zugabeeinrichtungen sind an der Innenseite der Seitenwand des Behälters angeordnet bzw. münden an der Innenfläche der Seitenwand des Behälters in den Behälter ein.

Für die Anbringung und Anordnung der Zugabeeinrichtungen bestehen unterschiedliche Möglichkeiten. Besonders vorteilhaft ist die Vorsehung mehrerer Zugabeeinrichtungen, die beispielsweise auf gleicher Höhe über dem Behälterboden bzw. dem Mischwerkzeug angeordnet sind und über den Umfang der Innenwand des Behälters vorzugsweise gleichmäßig verteilt sind.

Eine weitere Möglichkeit besteht darin, die einzelnen Zugabeeinrichtungen in einer geraden vertikalen Reihe oder einer schräg nach oben verlaufenden Reihe übereinander, gegebenenfalls versetzt oder spiralförmig, anzuordnen. Auch können die Zugabeeinrichtungen, insbesondere statistisch oder gleichmäßig, im Behälter verteilt sein oder es kann nur eine einzige Zugabeeinrichtungen vorgesehen sein.

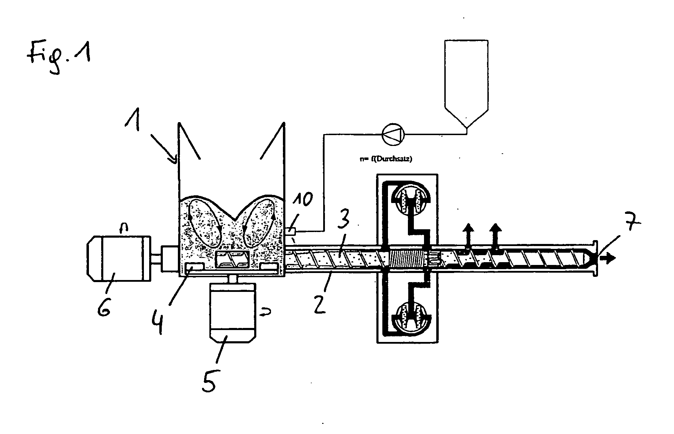

Die Zugabeeinrichtungen sind im Behälter so angeordnet, dass sie, insbesondere ständig und permanent, unterhalb des Niveaus des im Behälter befindlichen Materials liegen, sodass die Zugabe der Zusatzstoffe ausschließlich direkt in die Menge der rotierenden Materialteilchen erfolgt. Zumeist wird durch die Bewegung der Materialteilchen im Inneren des Behälters eine Mischthrombe ausgebildet, die schematisch auch in Fig. 1 dargestellt ist. Der Rand bzw. das oberste Niveau der Mischthrombe sollte, vorteilhafterweise während des gesamten Verfahrens, oberhalb der Zugabeeinrichtungen liegen.

Die Zugabeeinrichtungen sind vorteilhafterweise in Höhe des mittleren Drittelbereichs des Füllstandes des Materials im Behälter bzw. der Mischthrombe angeordnet, wodurch eine gleichmäßige Dispergierung der Zusatzstoffe auf die Materialteichen erfolgt.

Insbesondere für sehr hochviskose Zusatzstoffe ist es vorteilhaft, wenn die Zugabeeinrichtungen in demjenigen Bereich bzw. in derjenigen Höhe der Innenseite des Behälters angeordnet sind, auf den bzw. der die bewegten bzw. rotierenden Materialteilchen den höchsten Druck ausüben. Dadurch wird eine gute Verteilung des Materials sichergestellt. Dieser Bereich bzw. der vom Material auf die Seitenwand ausgeübte Druck wird in Abhängigkeit von der Drehzahl, der Art, Anzahl und Form der Mischwerkzeuge bestimmt.

Die Zugabeeinrichtungen können als einfache Zugabestutzen oder Zugabeöffnungen in der Seitenwand des Behälters ausgebildet sein oder auch in Form von Zugabedüsen ausgestaltet sein. Die Dosierung bzw. Beschickung der Zusatzstoffe erfolgt vorteilhafterweise über Dosierpumpen, beispielsweise Zahnradpumpen oder Membranpumpen. Diese steuern die Menge der zugegebenen Zusatzstoffe. Da, wie beschrieben, die gesamten Zusatzstoffe direkt in die Materialteilchen eingebracht werden können, kann sehr exakt und verlustfrei dosiert werden. Verluste durch Ablagerungen od. dgl. sind weitgehend ausgeschlossen.

Um die Bewegung der Materialteilchen im Inneren des Behälters nicht zu stören, ist es vorteilhaft, wenn die Zugabeeinrichtungen bündig mit der Innenwand des Behälters abschließen und nicht ins Innere des Behälters hineinragen bzw. abstehen.

Meist tritt der Zusatzstoff, der in das Material eingebracht werden soll, in Tröpfchenform oder in pastöser Form aus den Zugabeeinrichtungen aus. Die Materialteilchen bewegen sich durch die durch die Mischwerkzeuge erzwungene Bewegung entlang der Innenwand des Behälters, berühren diese und reiben an dieser. Dadurch werden durch die Materialteilchen die durch die Zugabeeinrichtung gerade austretenden Zusatzstoffe unmittelbar und direkt mitgerissen und verteilen sich umso besser in der Mischung.

Für manche Zusatzstoffe kann eine Benetzung der Behälterwand durch die Zusatzstoffe erwünscht sein, um dadurch eine bessere Dispergierung der Zusatzstoffe mit den Materialteilchen zu bewirken. Um dies zu ermöglichen, wird eine zusätzliche separate Heizvorrichtung vorgesehen , die lediglich die Innenseite der Seitenwand des Behälters bzw. die Behälterseitenwand erwärmt. Diese Heizeinrichtung ist unabhängig von temperierbaren bzw. beheizbaren Mischwerkzeugen oder weiteren Heizvorrichtungen zur Erwärmung des Materials im Behälter. Auf diese Weise verringert sich die Viskosität der Zusatzstoffe, wodurch die Fließfähigkeit der Zusatzstoffe verbessert wird und die Innenwand des Behälters besser benetzt ist. Dadurch ist eine noch bessere Verteilung der Zusatzstoffe auf die Materialteilchen gegeben.

Um Verstopfungen von pastösen oder hochviskosen Zusatzstoffen zu vermeiden, sind auch die Zugabeeinrichtungen selbst sowie deren Zuleitungen oder Vorratsbehälter beheizbar. Auf diese Weise können beispielsweise bei Raumtemperatur feste bzw. pastöse Wachse in ausreichend fluidisierter Form zugegeben werden. Allenfalls kann auch der Druck, der auf die Zusatzstoffe ausgeübt wird bzw. mit dem die Zusatzstoffe zugeführt werden, entsprechend eingestellt, insbesondere in entsprechender Höhe gewählt, werden, um höherviskose Zusatzstoffe einbringen zu können.

Grundsätzlich ist es vorteilhaft, das Material bei erhöhter Temperatur zu behandeln bzw. die Temperatur des Materials eher höher zu halten, da dadurch die Viskositäten der Zusatzstoffe herabgesetzt werden und eine bessere Verteilung und homogenere Dispergierung der Materialteichen erfolgt.

Weiters kann es vorteilhaft sein, eine Benetzung der Innenwand des Behälters durch die Zusatzstoffe zu vermeiden bzw. zu verringern. Dies kann beispielsweise durch besondere Beschichtungen oder auch durch besondere Prägungen der Behälterwand erfolgen. Auf diese Weise können sich die auf der Behälterinnenwand anhaftenden Zusatzstoff-Tröpfchen besser von der Seitenwand lösen und leichter vom Material bzw. von den bewegenden Materialteilchen mitgenommen werden und eine Benetzung der Behälterwand unterbleibt.

Weitere Vorteile und Ausgestaltungen der Erfindung ergeben sich aus der Beschreibung und der beiliegenden Zeichnung.

Die Erfindung ist anhand von Ausführungsbeispielen in der Zeichnung schematisch dargestellt und wird im Folgenden unter Bezugnahme auf die Zeichnung beispielsweise beschrieben.

Fig. 1 zeigt eine schematische Ansicht einer erfindungsgemäßen Vorrichtung.

In Fig. 1 ist eine erfindungsgemäße Vorrichtung in schematischer Schnittansicht dargestellt. Derartige Vorrichtungen sind aus dem Stand der Technik in verschiedenen Ausführungsformen bekannt.

Die in Fig.1 dargestellte Vorrichtung in Form eines Schneidverdichters weist einen Aufnahmebehälter 1 auf, in dessen Bodenbereich ein um eine etwa vertikale Achse verdrehbares Zerkleinerungs- bzw. Mischwerkzeug 4 vorgesehen ist, das von einem Antriebsmotor 5 angetrieben wird. Auf der Höhe dieses Zerkleinerungs- und Mischwerkzeugs 4 ist in der Seitenwand des Aufnahmebehälters 1 eine Öffnung vorgesehen, an die das Gehäuse 2 eines Schneckenextruders angeschlossen ist. Im Gehäuse 2 befindet sich eine Extruderschnecke 3, die von einem Antriebsmotor 6 angetrieben wird. Das durch den Schneckenextruder geförderte, zerkleinerte und gemischte Material, insbesondere ein Kunststoffgut, tritt durch die Austrittsöffnung 7 aus dem Schneckengehäuse aus. Bei der Verarbeitung von Kunststoffmaterial findet ein Aufschmelzen bzw. Plastifizieren des Materials erst im Extruder statt. Der Behälter 1 kann auch mit Vakuum beaufschlagt werden.

Im Aufnahmebehälter 1 wird das zu behandelnde Material vorgelegt. Das Material liegt im Aufnahmebehälter 1 in stückiger bzw. teilchenförmiger Form vor und hat damit im Verhältnis zu seinem Volumen eine große Oberfläche. Das Material kann beispielsweise thermoplastisches Kunststoffmaterial in Form von Flakes, Granulaten, Folienabfällen od. dgl. sein. Auch denkbar sind Holzfasern, Zeitungspapier od. dgl.. Durch die durch das Mischwerkzeug 4 verursachte stete dynamische Bewegung bzw. Rotation der Materialteilchen im Aufnahmebehälter 1 erfolgt eine Durchmischung der einzelnen Teilchen, sowie gegebenenfalls, je nach Ausbildung des Mischwerkzeuges 4, auch eine Zerkleinerung und/oder Vorverdichtung und gegebenenfalls auch eine Erwärmung des Materials bzw. eine Trocknung bzw. Kristallisation. Die Bewegung der Materialteilchen im Aufnahmebehälter 1 dient, insbesondere bei Kunststoffmaterialien, dazu, dass bei Erwärmung die einzelnen Kunststoffteilchen nicht zusammenkleben und dass der stückige Charakter des Materials erhalten bleibt.

Weiters ist im unteren Bereich der Seitenwand des Behälters 1 eine Zugabeeinrichtung 10 in Form eines Zugabestutzens vorgesehen, der über eine Öffnung in den Behälter 1 einmündet, wobei die Öffnung bündig mit der Innenfläche der Seitenwand abschließt und kein Teil der Zugabeeinrichtung 10 ins Innere des Behälters vorsteht. Über diese Zugabeeinrichtung 10 können ein oder mehrere Zusatzstoffe bzw. Beschichtungsstoffe in den Aufnahmebehälter 1 zudosiert werden.

Die Zugabeeinrichtung 10 ist so ausgestaltet, dass sie für die Zugabe von bzw. nicht-trocken-teilchenförmigen bzw. nicht-trocken-pulverförmigen bzw. nicht-trockengranulatförmigen bzw. nicht-trocken-kristallinen Zusatzstoffen geeignet ist. Trockene pulver- oder granulatförmige Zusatzstoffe, wie beispielsweise Pigmente, Füllstoffe od. dgl., werden zumeist über einfache Zugabetrichter von oben zugegeben. Über die Zugabeeinrichtung 10 werden somit pumpfähige dünn- oder zähflüssige, feste, halbfeste oder pastöse Zusatzstoffe, insbesondere von höherer Viskosität, zudosiert. Die Zugabeeinrichtung 10 ist beispielsweise geeignet, dünnflüssige Zusatzstoffe, wie Weichmacher, Peroxide, etc. zähflüssige Zusätze oder auch pastöse, eher festere Zusatzstoffe mit cremeartiger oder teigiger Konsistenz, wie beispielsweise Fette oder Wachse oder auch Polymere zuzugeben. Unter den Begriff der festen Zusatzstoffe sind beispielsweise Wachse oder Fette zu subsumieren, die bei Raumtemperatur zwar formstabil, jedoch immer noch duktil und verformbar sind. Auch ursprünglich pulverförmige Zusätze bzw. Zuschlagstoffe, wie Pigmente, Füllstoffe od. dgl. können mittels einer Trägerlösung als Dispersion oder Aufschlämmung, allenfalls auch als Suspension oder Emulsion, auf diese Weise zugegeben werden.

Die Zugabeeinrichtung 10 ist gemäß Fig. 1 unterhalb des Niveaus der im Behälter 1 befindlichen, rotierenden Materialteilchen bzw. unterhalb des obersten Randes der Mischthrombe angeordnet. Die Zugabe der Zusatzstoffe erfolgt somit nicht von oben, beispielsweise durch Aufsprühen oder Zutropfen, sondern erfolgt durch die Seitenwand des Behälters 1. Dabei wird die Zugabeeinrichtung 10 bzw. die Zugabeöffnung immer von sich vorbeibewegendem Material überstrichen und die austretenden Zusatzstoffe werden mitgerissen und so auf die Materialteilchen aufgebracht und innerhalb der Materialteilchen dispergiert bzw. verteilt.

Die Dispergierung der Zusatzstoffe funktioniert umso besser, je größer die Oberflächen der Materialteilchen sind.

Die Zusatzstoffe, insbesondere reaktive Zusatzstoffe, werden je nach Verdünnungsgrad durch einen allfälligen Träger der Zusatzstoffe, in Mengen zwischen 0,01 und 20 Gew.-% zugegeben. Beispielsweise wird bei der Verwendung von PET-Flakes als Vorlagematerial eine Menge von 0,2 bis 0,6 % eines Zusatzstoffes aufgebracht.

Die maximale Menge, mit der die Zusatzstoffe eingesetzt werden sollten, ist diejenige Menge, die notwendig ist, um die Gesamtoberfläche des im Behälter 1 befindlichen Materials bzw. die Gesamtoberfläche der Materialteilchen zu benetzen.

Je nach der Art des eingesetzten Zusatzstoffes und dessen Reaktivität findet eine Reaktion des Zusatzstoffes mit dem Material unter Umständen erst im Extruder bzw. in der Schmelze statt.

Das Material wird abschließend im Extruder vollständig aufgeschmolzen und wenn nötig filtriert und/oder entgast.

Das erfindungsgemäße Verfahren kann einstufig geführt werden, aber auch in einen zwei- oder mehrstufigen Prozess eingebunden sein. Die Zusatzstoffe werden dabei vorteilhafterweise bereits in der ersten Stufe, in einem vorgeschalteten Vorbehandlungsbehälter bzw. einem ersten Aufnahmebehälter 1 zugegeben. Die Zugabeeinrichtungen sind zu diesem Zweck in diesem Vorbehandlungsbehälter angeordnet. Die weitere Behandlung des Materials und/oder die Zugabe weiterer Zusatzstoffe oder eine allfällige Trocknung bzw. Kristallisation erfolgen dann in weiteren Behältern 1.

## Patentansprüche

1. Verfahren zur Einbringung bzw. Zugabe von nicht-trocken-teilchenförmigen, insbesondere nicht-trocken-pulverförmigen, Zusatzstoffen bzw. Beschichtungsstoffen mit flüssiger, fester, halbfester oder pastöser Konsistenz, gegebenenfalls in suspendierter oder emulgierter Form, insbesondere mit höherer Viskosität, wie beispielsweise Peroxiden, Fetten, Wachsen, IV-Verbesserern, Polymeren od. dgl., zu einem, in einem Aufnahmebehälter bzw. Schneidverdichter (1) bewegten, gemischten und gegebenenfalls erwärmten und zerkleinerten, stückig bzw. teilchenförmig vorliegenden Material, insbesondere Polymerteilchen bzw. -flakes, Holzfasern, Papierschnitzein od. dgl., wobei die Zusatzstoffe unterhalb des Niveaus des im Behälter (1)
befindliche Materials bzw. der Materialteilchen zugegeben werden, **dadurch gekennzeichnet, dass** vor und/oder während der Zugabe der Zusatzstoffe, unabhängig von der Temperatur des im Behälter (1) befindlichen Materials, ausschließlich die Innenseite bzw. die Seitenwand des Behälters (1) *und die Zugabeeinrichtungen (10) für die Zusatzstoffe und*/*oder deren Zuleitungen bzw. Vorratsbehälter,* zusätzlich und separat erwärmt *werden,* um die Viskosität der zugeführten Zusatzstoffe zu vermindern und die Benetzung zu erhöhen.

2. Verfahren nach Anspruch 1, **dadurch gekennzeichnet, dass** die Zusatzstoffe in den mittleren Drittelbereich des Füllstandes des Materials im Behälter (1) bzw. einer durch die Rotation ausgebildeten Mischthrombe, zugegeben werden.

3. Verfahren nach Anspruch 1 oder 2, **dadurch gekennzeichnet, dass** die Zusatzstoffe, Insbesondere sehr hochviskose Zusatzstoffe, in denjenigen Bereich bzw. in derjenigen Höhe des Behälters (1) zugegeben werden, in dem die, insbesondere im Behälter (1) rotierenden, Matertalteilchen den höchsten Druck auf die Seitenwand des Behälters (1) ausüben.

4. Verfahren nach einem der Ansprüche 1 bis 3, **dadurch gekennzeichnet, dass** die Zusatzstoffe über eine oder mehrere, an der Innenseite der Seitenwand des Behälters (1) angeordnete bzw, durch die Seitenwand einmündende, insbesondere in gleicher Höhe umfänglich verteilte oder in einer Reihe übereinanderliegende, als Zugabeöffnungen oder Düsen ausgebildete, gegebenenfalls über Dosierpumpen, beispielsweise Zahnradpumpen oder Membranpumpen beschickte, Zugabeeinrichtung(en) (10) zugegeben werden.

5. Verfahren nach einem der Ansprüche 1 bis 4, **dadurch gekennzeichnet, dass** das Niveau der Matertaltellchen bzw. der durch die Bewegung ausgebildeten Mischthrombe im Behälter (1) so gehalten wird, dass es ständig oberhalb der Zugabeeinrichtung(en) (10) liegt.

6. Verfahren nach einem der Ansprüche 1 bis 5, **dadurch gekennzeichnet, dass** die Zusatzstoffe durch die bzw. mittels der an der Innenseite der Seitenwand des Behälters (1) und an den Zugabeeinrichtungen (10) entlang- bzw. vorbeistreifenden bzw. - rotierenden Materialteilchen mitgenommen bzw. eingebracht werden bzw. die Zusatzstoffe auf diese Weise auf die Meterialteilchen aufgebracht werden.

7. Verfahren nach einem der Ansprüche 1 bis 6, **dadurch gekennzeichnet, dass**
die Zusatzstoffe, insbesondere reaktive Zusatztoffe, je nach Verdünnungsgrad durch einen allfälligen Träger der Zusatzstoffe, in Mengen zwischen 0,01 und 20 Gew%, bezogen auf das Gesamtgewicht des Endproduktes, zugegeben werden und/oder
die Zusatzstoffe maximal bis zu einer Menge zugesetzt werden, die nötig ist, um die Gesamtoberttäche des im Behälter (1) befindlichen Materials bzw. der Materialteilchen zu benetzen.

8. Vorrichtung zur Durchführung des Verfahrens nach einem der Ansprüche 1 bis 7, mit zumindest einem Aufnahmebehälter bzw. Schneidverdichter (1), in dem zumindest ein, insbesondere um eine vertikale Achse drehbares, das zu behandelnde stückige bzw. teilchenförmige Material, insbesondere ein Kunststoffmeterial in Form von nicht geschmolzenen Polymerteilchen, Holzfasern, Papierschnitzeln od. dgl., bewegende bzw. im Rotation versetzende, mischende, erwärmende bzw. gegebenenfalls zerkleinernde, Mischwerkzeug (4) angeordnet ist, wobei zumindest eine Zugabeeinrichtung (10) für nicht-trocken-teilchenfärmiga Zusatzstoffe mit flüssiger, fester, halbfester oder pastöser Konsistenz, gegebenenfalls in suspendierter oder emulgierter Form, insbesondere mit höherer Viskosität, beispielsweise Peroxide, Fette, Wachse, IV-Verbesserer, Polymere od. dgl., vorgesehen ist, wobei die Zugabeeinrichtung (10) unterhalb des Niveaus des im Betrieb im Behälter (1) befindlichen Materials bzw. der Materialteilchen angeordnet ist,
**dadurch gekennzeichnet, dass** unabhängig von bzw. zusätzlich zu der Erwärmung des Materials durch beispielsweise die Mischwerkzeuge (4) oder weiteren das Material erwärmenden Erwärmungsvorrichtungen, zumindest eine separate Heizeinrichtung vorgesehen ist, mit der ausschließlich die Innenseite bzw. die Seitenwand des Behälters (1) und die Zugabeeinrichtungen (10) und/oder deren Zuleitungen bzw. Vorratsbehälter, separat beheizbar sind, um die Viskosität der zugegebenen Zusatzstoffe zu vermindern und die Benetzung an der Seitenwand des Behälters (1) zu erhöhen.

9. Vorrichtung nach Anspruch 8, **dadurch gekennzeichnet, dass** die Zugabeeinrichtungen (10) an der Innenseite der Seitenwand des Behälters (1) angeordnet sind bzw. in der Seitenwand des Behälters (1) in den Behälter (1) einmünden bzw. ausgebildet sind und/oder
dass die Zugabeeinrichtungen (10) auf gleicher Höhe über den Umfang der Innenwand des Behälters (1), vorzugsweise gleichmäßig, verteilt oder in einer Reihe übereinanderliegend, angeordnet sind.

10. Vorrichtung nach Anspruch 8 oder 9, **dadurch gekennzeichnet, dass** die Zugabeeinrichtungen (10) im Behälter (1) in einer Höhe bzw. in einem Abstand zum Boden bzw. zum Mischwerkzeug (4) angeordnet sind, in der/dem die Zugabeeinrichtungen (10) ständig unterhalb des verfahrensmäßig vorgegebenen Füllstandes der im Behälter (1) befindlichen bzw. rotierenden Materialteilchen bzw. des Niveaus der bei einer Bewegung bzw. Rotation der Materialteilchen ausgebildeten Mischthrombe liegen.

11. Vorrichtung nach einem der Ansprüche 8 bis 10, **dadurch gekennzeichnet, dass** die Zugabeeinrichtungen (10) in Höhe des mittleren Drittelbereichs des verfahrensmäßig vorgegebenen Füllstandes des Materials im Behälter (1) bzw. der Mischthrombe angeordnet sind.

12. Vorrichtung nach einem der Ansprüche 8 bis 11, **dadurch gekennzeichnet, dass** die Zugabeeinrichtungen (10) in demjenigen Bereich des Behälters (1) angeordnet sind, in dem die, vorzugsweise bewegten, Insbesondere im Behälter (1) rotierenden, Materialteilchen den höchsten Druck auf die Seitenwand des Behälters (1) ausüben.

13. Vorrichtung nach einem der Ansprüche 8 bis 12, **dadurch gekennzeichnet, dass** die Zugabeeinrichtungen (10) als Austrittsöffnungen oder Düsen ausgebildet sind und vorzugsweise mittels Dosierpumpen, beispielsweise Zahnradpumpen oder Membranpumpen, beschickbar sind und insbesondere so ausgebildet sind, dass die Zusatzstoffe in Tröpfchenform eindosierbar sind
und/oder
dass die Zugabeeinrichtungen (10) bündig mit der Innenwand des Behälters (1) abschließen und insbesondere nicht von der Innenseite des Behälters (1) ins Innere des Behälters (1) vor- bzw. abstehen.

14. Vorrichtung nach einem der Ansprüche 8 bis 13, **dadurch gekennzeichnet, dass**,
die Oberfläche der Innenseite des Behälters (1), insbesondere durch Aufbringung einer Anti-Haft-Beschichtung, einer Prägung, etc., als nicht benetzbare Oberfläche ausgebildet ist bzw. so ausgestaltet ist, dass die Benetzbarkeit minimiert bzw. keine Benetzung der Innenseite durch die zugegebenen Zusatzstoffe erfolgt.

15. Vorrichtung nach einem der Ansprüche 8 bis 14, **dadurch gekennzeichnet, dass** mehrere Aufnahmebehälter (1), insbesondere aufeinanderfolgend, vorgesehen sind und die Zugabeeinrichtungen (10) zumindest im ersten Aufnahmebehälter (1) angeordnet sind.

## Claims

1. A method for introducing or adding non-dry particulate, particularly non-dry powdered, additives or coating substances of liquid, solid, semisolid or paste-like consistency, optionally in a suspended or emulsified form, particularly of a higher viscosity, such as peroxides, lipids, waxes, IV improvers, polymers or the like, into a mixed and optionally heated and crushed, lumpy or particulate material, which is agitated in a receiving container or a cutter compactor (1), particularly polymer particles or flakes, wood fibres, paper scrap, etc., said additives being added below the level of the material or the material particles in the container (1),
**characterised in that** before and/or while the additives are added, independent of the temperature of the material in the container (1), only the interior surface and the lateral wall of the container (1) and the adding devices (10) for adding the additives and/or their conduits and reservoirs are additionally and separately heated in order to reduce the viscosity of the supplied additives and to improve the wetting.

2. The method according to claim 1, **characterised in that** the additives are added in the area of the central third of the material level in the container (1) or via a mixing vortex generated by the rotation.

3. The method according to claim 1 or claim 2, **characterised in that** the additives, particularly high viscosity additives, are added in the area or at the height of the container (1) where the material particles, which particularly rotate within said container (1), exert the highest pressure on the container's (1) lateral wall.

4. The method according to any one of the claims 1 to 3, **characterised in that** the additives are added by means of one or several adding device(s) (10) which are disposed on the interior surface of the lateral wall of the container (1) and extend through said lateral wall, are distributed particularly at the same height around the container's (1) circumference or in a line above one another, are formed as addition openings or nozzles, the additives being optionally supplied to them by means of dosing pumps, such as gear pumps or diaphragm pumps.

5. The method according to any one of the claims 1 to 4, **characterised in that** the level of the material particles or of the mixing vortex generated by their movement in the container (1) is maintained above the height of said adding device(s) (10) at all times.

6. The method according to any one of the claims 1 to 5, **characterised in that** the additives are carried on and introduced by means of the material particles which pass or are rotated along or sweep the interior surface of the lateral wall of the container (1) and along the adding devices (10) or **in that** the additives are applied to the material particles in this way.

7. The method according to any one of the claims 1 to 6, **characterised in that**, depending on their degree of dilution, the additives, particularly reactive additives, are added using an optional carrier in an amount of 0.01 to 20 % by weight based on the overall weight of the final product
and/or
**in that** the maximum amount of additives which is added to the container (1) corresponds to the amount required to wet the entire surface of the material or the material particles in the container (1).

8. A device for carrying out the method according to any one of the claims 1 to 7, including at least one receiving container or cutter compactor (1) in which at least one mixing tool (4) which particularly rotates around a vertical axis and moves or rotates, mixes, heats and optionally crushes the lumpy or particulate material to be treated, particularly plastic material in the form of non-molten polymer particles, wood fibres, paper shreds and the like, at least one adding device (10) for non-dry particulate additives of a liquid, solid, semisolid or pasty consistency, particularly having a higher viscosity, such as peroxides, lipids, waxes, IV improvers, polymers or the like, being provided, said adding device (10) being provided below the level of the material or the material particles in the container (1) during operation,
**characterised in that**, independent of and in addition to the heating of the material, for example, by the mixing tools (4) or other heating devices heating the material, at least one separate heating device is provided, which separately heats only the interior surface or the lateral wall of the container (1) and the adding devices (10) and/or the conduits and the reservoirs of said adding devices in order to reduce the viscosity of the added additives and improve the wetting of the container's (1) lateral wall.

9. The device according to claim 8, **characterised in that** the adding devices (10) are disposed on the interior surface of the lateral wall of the container (1) and extend through said lateral wall of the container (1) leading into said container (1) and/or
**in that** the adding devices (10) are distributed at the same height around the circumference of the lateral wall of the container (1), preferably at equal intervals, or in a line above one another.

10. The device according to claim 8 or claim 9, **characterised in that** the adding devices (10) are disposed in the container (1) at a height or at a distance from the bottom or the mixing tool (4), so that the adding devices (10) are below the level of the material particles present or rotating in the container (1), as defined according to the method, or below the level of the mixing vortex generated by the particles' movement or rotation at all times.

11. The device according to any one of the claims 8 to 10, **characterised in that** the adding devices (10) are disposed at the height of the central third of the level of the material in the container (1) or of the mixing vortex, as defined according to the method.

12. The device according to any one of the claims 8 to 11, **characterised in that** the adding devices (10) are disposed in the area of the container (1) in which the material particles, which are preferably moved and particularly rotated within said container (1), exert the highest pressure on the container's (1) lateral wall.

13. The device according to any one of the claims 8 to 12, **characterised in that** the adding devices (10) are formed as outlet openings or nozzles and the additives are preferably supplied to them by means of dosing pumps, such as gear pumps or diaphragm pumps, and the adding devices (10) are preferably formed so that the additives may be added in droplets
and/or
**in that** the adding devices (10) are flush mounted to the interior wall of the container (1) and particularly do not project into the interior of the container (1) from the interior surface of the container (1).

14. The device according to any one of the claims 8 to 13, **characterised in that** the interior surface of the container (1) is formed as a non-wettable surface, particularly by applying an nonstick coating, an embossment, etc., in order to reduce its wettability or to make sure that the interior surface is not wetted at all by the added additives.

15. The device according to any one of the claims 8 to 14, **characterised in that** several receiving containers (1) are provided, particularly one after the other, and **in that** the adding devices (10) are disposed at least in the first receiving container (1).

## Revendications

1. Procédé pour l'introduction ou l'ajout d'additifs ou d'agents d'enrobage non-secs particulaires, notamment d'additifs ou d'agents d'enrobage non-secs en poudre, à une consistance liquide, solide, demisolide ou pâteuse, éventuellement en suspension ou en émulsion, notamment à une viscosité élevée, comme, par exemple, de peroxides, lipides, cires, d'agents améliorant la VI, de polymères etc., dans une matière en morceaux ou en particules mélangée et éventuellement chauffée et broyée, ladite matière, notamment des particules ou des flacons polymères, des fibres ligneuses, des rognures de papier, etc., étant agitée dans un récipient ou coupeur-compacteur (1), lesdits additives étant ajoutés au-dessous du niveau de matière ou de matière particulaire dans le récipient (1), **caractérisé en ce que** avant ou/et pendant les additives sont ajoutées et indépendamment de la température de la matière dans le récipient (10), seulement la surface intérieure ou la paroi latérale du récipient (1) et les dispositifs d'ajout (10) pour ajouter les additifs et/ou leurs conduites et réservoirs sont chauffés supplémentairement et séparément pour réduire la viscosité des additifs fournis et d'augmenter le mouillage.

2. Procédé selon la revendication 1, **caractérisé en ce que** les additifs sont ajoutés dans le tiers central du niveau de la matière dans le récipient (1) ou à travers un tourbillon mélangeur généré par la rotation.

3. Procédé selon la revendication 1 ou 2, **caractérisé en ce que** les additifs, notamment des additifs à haute viscosité, sont ajoutés dans la partie ou à la hauteur du récipient (1) où les particules de la matière, qui se trouvent notamment en rotation dans le récipient (1), exercent la pression la plus haute sur la parois latérale du récipient (1).

4. Procédé selon l'une des revendications 1 à 3, **caractérisé en ce que** les additifs sont ajoutés par un ou plusieurs dispositif(s) d'ajout (10) qui sont disposés sur la surface intérieure de la paroi latérale du récipient (1) et s'étendent à travers ladite paroi latérale, étant répartis le long de la circonférence du récipient, notamment à la même hauteur, ou sur une ligne l'un au dessous de l'autre, étant formés par des orifices d'ajout ou des tuyères, auxquels les additifs sont fournis par des pompes de dosage, par exemple par des pompes à engrenages ou des pompes à diaphragme, selon le cas.

5. Procédé selon l'une des revendications 1 à 4, **caractérisé en ce que** le niveau des particules de matière ou du tourbillon mélangeur généré par le mouvement des particules dans le récipient (1) est toujours maintenu au-dessus de la hauteur du dispositif d'ajout/des dispositifs d'ajout (10).

6. Procédé selon l'une des revendications 1 à 5, **caractérisé en ce que** les additifs sont emportés et introduits par les particules de matière qui passent ou tournent le long ou passent sur la surface intérieure de la paroi latérale du récipient (1) et le long des dispositifs d'ajout (10) ou **en ce que** les additifs sont appliqués sur les particules de matière dans cette façon.

7. Procédé selon l'une des revendications 1 à 6, **caractérisé en ce que**, selon le degré de dilution les additifs, ceux-ci, notamment les additifs réactifs, sont ajoutés en utilisant un porteur facultatif, à un taux de 0,01 à 20 % en poids par rapport au poids total du produit final
et/ou
**en ce que** le taux maximal d'additifs ajouté dans le récipient (1) correspond au taux nécessaire pour le mouillage de la surface entière de la matière ou des particules de matière dans le récipient (1).

8. Dispositif pour la réalisation d'un procédé selon l'une des revendications 1 à 7, comprenant au moins un récipient ou coupeur-compacteur (1) dans lequel au moins un outil de mélange (4) qui notamment tourne autour une axe verticale et agite ou tourne, mélange, chauffe et éventuellement broie la matière en morceaux ou en particules à traiter, notamment de la matière plastique en forme de particules polymères non-fondues, des fibres ligneuses, des rognures de papier, etc., au moins un dispositif d'ajout (10) pour des additifs non-secs particulaires à une consistance liquide, solide, demisolide ou pâteuse, notamment à une viscosité élevée, comme, par exemple, de peroxides, lipides, cires, d'agents améliorant la VI, de polymères etc., étant fourni, ledit dispositif d'ajout (10) étant fournis au-dessous du niveau de la matière ou des particules de matière dans le récipient (1), quand le dispositif est en service,
**caractérisé en ce qu'**au moins un dispositif de chauffage particulier est prévu, indépendant de et comme un supplémentaire au chauffage de la matière par les outils de mélange (4), par exemple, ou d'autres dispositifs de chauffage chauffant la matière, ledit dispositif de chauffage chauffant séparément seulement la surface intérieure ou la paroi latérale du récipient (1) et les dispositifs d'ajout (10) et/ou les conduites et les réservoirs desdits dispositifs d'ajout pour réduire la viscosité des additifs fournis et améliorer le mouillage de la paroi latérale du récipient (1).

9. Dispositif selon la revendication 8, **caractérisé en ce que** les dispositifs d'ajout (10) sont disposés sur la surface intérieure de la paroi latérale du récipient (1) et s'étendent à travers ladite paroi latérale du récipient (1) s'ouvrant vers son intérieur et/ou **en ce que** les dispositifs d'ajout (10) sont répartis, de préférence régulièrement, à la même hauteur le long de la circonférence du récipient ou sur une ligne l'un au dessous de l'autre.

10. Dispositif selon la revendication 8 ou 9, **caractérisé en ce que** les dispositifs d'ajout (10) sont disposés dans le récipient (1) à une hauteur ou à une distance du fond ou de l'outil de mélange (4) pour que les dispositifs d'ajout (10) se trouvent toujours au-dessous le niveau des particules de matière présentes ou tournant dans le récipient (1), comme défini selon le procédé, ou au-dessous le niveau du tourbillon mélangeur généré par le mouvement ou la rotation des particules.

11. Dispositif selon l'une des revendications 8 à 10, **caractérisé en ce que** les dispositifs d'ajout (10) sont disposés à la hauteur du tiers central du niveau de la matière dans le récipient ou du tourbillon mélangeur, comme défini selon le procédé.

12. Dispositif selon l'une des revendications 8 à 11, **caractérisé en ce que** les dispositifs d'ajout (10) sont disposés dans la partie du récipient (1) où les particules de matière, qui, de préférence, sont agitées et notamment tournées dans le récipient (1), exercent la pression la plus haute sur la parois latérale du récipient (1).

13. Dispositif selon l'une des revendications 8 à 12, **caractérisé en ce que** les dispositifs d'ajout (10) sont formés comme des orifices de sortie ou des tuyères et **en ce que** les additifs, de préférence, y sont fournis par des pompes de dosage, par exemple par des pompes à engrenages ou des pompes à diaphragme, et **en ce que** les dispositifs d'ajout (10) sont, de préférence, formés ainsi que les additifs peuvent être ajoutés goutte à goutte
et/ou
**en ce que** les dispositifs d'ajout (10) sont à fleur de la paroi intérieure du récipient (1) et, notamment, ne saillissent pas vers l'intérieur du récipient (1) de la surface intérieure du récipient (1).

14. Dispositif selon l'une des revendications 8 à 13, **caractérisé en ce que** la surface intérieure du récipient (1) est formée comme une surface non-mouillable, notamment par l'application d'un enrobage antiadhésif, d'une gravure, etc., pour réduire sa mouillabilité ou pour assurer que ladite surface intérieure n'est pas du tout mouillée par les additifs ajoutés.

15. Dispositif selon l'une des revendications 8 à 14, **caractérisé en ce que** plusieurs récipients (1) sont prévus, notamment l'un après l'autre, et **en ce que** les dispositifs d'ajout (10) sont disposés au moins dans le premier récipient (1).
